# EUROPÄISCHE PATENTANMELDUNG

(11) **EP 0 873 719 A2**
(43) Veröffentlichungstag der Anmeldung: **28.10.1998**
(21) Anmeldenummer: 98102176.9
(22) Anmeldetag: 09.02.1998
(51) Int. Cl.: A61B 17/22, A61M 29/02

(54) **Vorrichtung mit einem therapeutischen Katheter**

(30) Priorität: 26.04.1997 DE 19717790
(71) Anmelder: CONVERGENZA AG, FL-9490 Vaduz (LI)
(72) Erfinder: Tiedtke, Hans Jürgen, 52249 Eschweiler (DE); Lussem, Horst, 52351 Düren (DE)
(74) Vertreter: Vetter, Ewald Otto, Dipl.-Ing.

(57) **Zusammenfassung**

Vorrichtung mit einem pneumatischen oder hydraulischen oder mechanischen therapeutischen Katheter. Die zur Behandlung von inneren Gefäßen eines Patienten erforderliche Therapie-Energie wird durch das gleiche Fluid oder das gleiche Element vom proximalen Endabschnitt zum distalen Endabschnitt des Katheters übertragen, wie Informationen vom distalen Endabschnitt zum proximalen Endabschnitt des Katheters übertragen werden. Die Informationen geben Auskunft über den Zustand des Gefäßes und wie das Therapie-Fluid oder das Therapie-Element des Katheters auf das Gefäß wirkt.

## Beschreibung

Die Erfindung betrifft eine Vorrichtung mit einem therapeutischen Katheter gemäß dem Oberbegriff von Anspruch 1 oder Anspruch 6.

Aus der EP-A-0 527 312 (entspricht DE 41 26 886 A1) ist ein therapeutischer Katheter bekannt, welcher mindestens zwei in Längsrichtung sich durch ihn hindurcherstreckende Kanäle oder Lumen hat. Das eine Lumen ist ein Vorlaufkanal zur Zuführung von Flüssigkeit mit hohem Druck, beispielsweise 75 bar, vom nahen oder proximalen Ende zum fernen oder distalen Ende des Katheters. Der Katheter hat an seinem distalen Ende einen offenen Strömungsweg, auf welchem die Flüssigkeit in Form eines scharfen Strahles Teile von einem Körpergewebe in einem Patienten abtrennen und in das andere Lumen fördern kann, welches als Rücklaufkanal dient und die Flüssigkeit zusammen mit dem abgetrennten Gewebematerial zum proximalen Ende des Katheters transportiert. Ein ähnlicher therapeutischer Katheter ist auch aus der veröffentlichten Patentanmeldung DE 42 01 992 A1 bekannt. Andere bekannte therapeutische Katheter, wie beispielsweise aus der US 5 380 273 bekannt, haben an ihrem distalen Ende einen oder auch zwei Expansionselemente, sogenannte Dilatationsballone, mit welchen in einem Patienten Blutbahnen erweitert oder blockiert werden können. Ferner sind aus der US 5 092 872 und den offengelegten deutschen Patentanmeldungen DE 38 01 318 A1, DE 38 28 478 A1 und DE 43 23 756 A1 mechanische Katheter mit einem rotierenden Werkzeug an ihrem distalen Endabschnitt zum Abtragen von Material in Gefäßen eines Patienten bekannt. Ferner zeigt die PCT-WO 89/09029 einen mechanischen Katheter mit schwenkbaren Werkzeugen am distalen Katheterende.

Beim Einführen eines Katheters in einen Patienten kann der Arzt die Position des Katheters auf einem Röntgenbildschirm beobachten. Auf dem Röntgenbildschirm ist jedoch das Gewebe oder der Zustand des Gewebes nicht oder nicht deutlich erkennbar. Der Arzt ist deshalb nicht in der Lage, während der Therapie den Therapieerfolg online zu überprüfen. Er bekommt keine Rückmeldung, ob beispielsweise ein Gefäßverschluß ausreichend beseitigt worden ist. Er ist zu einem iterativen Vorgehen 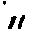 Therapie, Kontrolle, Therapie usw." gezwungen, wobei der Arzt bei der Festlegung der Kontrollintervalle auf seine Erfahrung und sein Gefühl angewiesen ist. Diese Vorgehensweise führt zu häufigen Kontroll-Aniographien, die den Patienten mit Strahlung und Kontrastmittel belasten.

Zusammenfassend bedeutet dies, daß bei der Verwendung von therapeutischen Kathetern der vorgenannten Arten in Kombination mit Röntgensichtgeräten der Anwender nur Informationen über die Lage des Katheters im Patienten erhält, jedoch die eigentliche therapeutische Funktion nicht sichtbar ist.

Aus der EP 0 485 133 A1 ist ein hydrodynamischer Thrombektomiekatheter bekannt, bei welchem über ein Kanalsystem Fluid zur Behandlung von Patientengewebe und über ein davon getrenntes System Ultraschall zur Katheterspitze zur optischen Kontrolle des Katheterbetriebes geleitet wird. Der Therapiekanal für das Fluid und der Diagnosekanal für die Ultraschalldiagnose sind getrennt und unabhängig voneinander.

Bei Kathetern, bei welchen Laserstrahlen oder Ultraschallwellen zur Behandlung von Patientengewebe verwendet werden, kann die Funktion der Katheterspitze in einem Patientengewebe auf einfache Weise durch reflektierende Laserstrahlen oder Ultraschallwellen überwacht werden. Solche Katheter sind beispielsweise aus folgenden Dokumenten bekannt: Veröffentlichung des Artikels 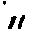 Laser-induced Shockwave Lithotripsy with Microsecond Laser Pulses" in der Zeitschrift Laser und Optoelektronik 20(4)/1988 von R. Engelhardt, W. Meyer, S. Thomas, P. Oehlert; veröffentlichte Patentanmeldungen DE 43 22 955 A1 und DE 195 22 310 A1; Patentschrift DE 42 40 182 C2, US-Patent 5 104 392 und EP 0 582 766 A1. Ferner ist aus der DE 44 37 578 A1 ein therapeutischer Katheter zur Behandlung von Patientengewebe mit Laserlicht bekannt, welcher einen zusätzlichen internen Hohlkanal aufweist, durch welchen ein Laserstrahl für Diagnosezwecke übertragen wird. Ferner sind aus der EP 0 629 380 A1 mehrere therapeutische Katheter bekannt: ein Katheter zur Beseitigung von Stenosen durch Laserbestrahlung, ein Katheter mit einem expandierbaren Ballon am distalen Katheterende zur Beseitung von Stenosen, und ein Katheter zur Behandlung von Patientengefäßen mit Ultraviolettlicht zur Verhinderung von Neubildungen von Stenosen in Blutgefäßen des Patienten. Bei diesen zuletztgenannten Kathetern sind keine Diagnosemöglichkeiten vorgesehen.

Durch die Erfindung soll die Aufgabe gelöst werden, bei Kathetern, welche zur therapeutischen Behandlung von Patientengefäßen Fluidkanäle und/oder mechanische Werkzeuge aufweisen, eine Möglichkeit zu schaffen, durch welche auf einfache Weise ein Signal erzeugt werden kann, welches dem Anwender Hinweise auf die therapeutische Wirkung seiner Arbeit mit dem Katheter in einem Patienten gibt.

Diese Aufgabe wird gemäß der Erfindung durch die kennzeichnenden Merkmale von Anspruch 1, Anspruch 8 und Anspruch 9 gelöst.

Der Grundgedanke der Erfindung ist folgender: Bei Anwendung von therapeutischen Kathetern, bei welchen die Energie in Form von unter Druck stehendem flüssigem oder gasförmigem Fluid oder in Form von mechanischer Energie vom proximalen Endabschnitt zum distalen Endabschnitt, insbesondere zur Katheterspitze, des Katheters transportiert und dort zur Therapie in einem Patienten benutzt wird, wird die Energie durch Rückwirkung von dem Patientengewebe verändert. Oder mit anderen Worten ausgedrückt, die Größe und die Art der Energie, welche an der Katheterspitze an die Umgebung abgegeben wird, ändert sich durch Rückwirkungen von der Umgebung auf diese Energie. Bei einem unter Druck stehenden gasförmigen oder flüssigen Fluid zum Abtragen von Material in Patientengefäßen ändert sich der Druck des Fluides in Abhängigkeit vom Strömungswiderstand des Fluides zwischen dem distalen Endabschnitt des Katheters und seiner Umgebung, welche durch das Patientengewebe gebildet ist. Ferner entstehen im Fluidstrom Geräusche und damit Schallwellen in Abhängigkeit davon, ob das Fluid auf ein Patientengewebe auftrifft und/oder Patientengewebe abreißt und/oder abgerissenes oder abgeschnittenes Patientengewebe vom Fluid in den Fluidkanal gezogen wird. Diese Druckänderungen und/oder am distalen Endabschnitt des Katheters auftretenden Geräusche oder Schallwellen werden gemäß der Erfindung am proximalen Endabschnitt des Katheters durch einen Sensor oder eine Meßvorrichtung ermittelt und für eine Bedienungsperson des Katheters akustisch hörbar oder visuell sichtbar gemacht. Dadurch kann die Bedienungsperson anhand dieser akustischen oder optischen Signale erkennen, ob das Fluid, und in welcher Weise das Fluid, in einem Patienten auf ein Patientengewebe wirkt. In analoger Weise wird durch die Erfindung bei Anwendung auf mechanische Katheter bei der therapeutischen Behandlung von Patientengefäßen das Drehmoment und/oder Geräuschentwicklungen einer Antriebswelle gemessen, welche ein rotierendes Werkzeug am distalen Katheterende antreibt. Aus diesen Messungen wird wie vorstehend beschrieben ein akustisches und/oder visuell sichtbares Signal oder Meßergebnis erzeugt, anhand welcher eine Bedienungsperson die Wirkung des rotierenden Werkzeuges auf ein Patientengefäß in einem Patienten erkennen und beurteilen kann. Durch die Erfindung werden diese Änderungen der Energie in Form von Druckänderungen, Schallerzeugung oder Schalländerungen oder Drehmoment und Drehmomentänderungen, die am distalen Endabschnitt des Katheters auftreten, am proximalen Endabschnitt des Katheters ermittelt oder gemessen, wobei diese Messung am gleichen Fluid oder am gleichen mechanischen Element am proximalen Endabschnitt des Katheters ausgeführt wird, welches die Energie zum distalen Endabschnitt des Katheters fördert. Damit wird gemäß der Erfindung für die Therapie eines Patienten der gleiche Kanal und das gleiche Fluid oder das gleiche mechanische Element verwendet wie für die Signalgewinnung. Durch Anschließen eines Sensors oder Meßelements an den Fluidkanal oder das mechanische Antriebselement am proximalen Endabschnitt des Katheters werden die vorgenannten Änderungen registriert und dem Benutzer sichtbar und/oder hörbar gemacht. Das Behandlungsmedium 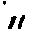 Fluid" oder 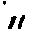 Antriebselement" hat somit eine Doppelfunktion, indem es sowohl zur Therapie und Energieübertragung vom proximalen Endabschnitt zum distalen Endabschnitt des Katheters dient, als auch in umgekehrter Richtung als Informationsweg vom distalen Endabschnitt zum proximalen Endabschnitt des Katheters. Für die Informationsübertragung ist kein Informationsweg zusätzlich zum Therapieweg erforderlich.

Weitere Merkmale der Erfindung sind in den Unteransprüchen enthalten.

Die Erfindung wird im folgenden mit Bezug auf die Zeichnungen anhand von bevorzugten Ausführungsformen als Beispiele beschrieben. In den Zeichnungen zeigen
- Fig. 1: schematisch eine Vorrichtung mit einem Ballon-Katheter zur therapeutischen Behandlung,
- Fig. 2: schematisch eine Vorrichtung mit einem mechanischen Katheter mit rotierender Welle,
- Fig. 3: schematisch eine Vorrichtung mit einem Hochdruck-Fluid-Katheter zum Entfernen von Gefäßteilchen in einem Patienten, mit akustischen und optischen Anzeigemitteln für Druck und/oder Schall und/oder Durchflußrate des Fluides im Katheter.

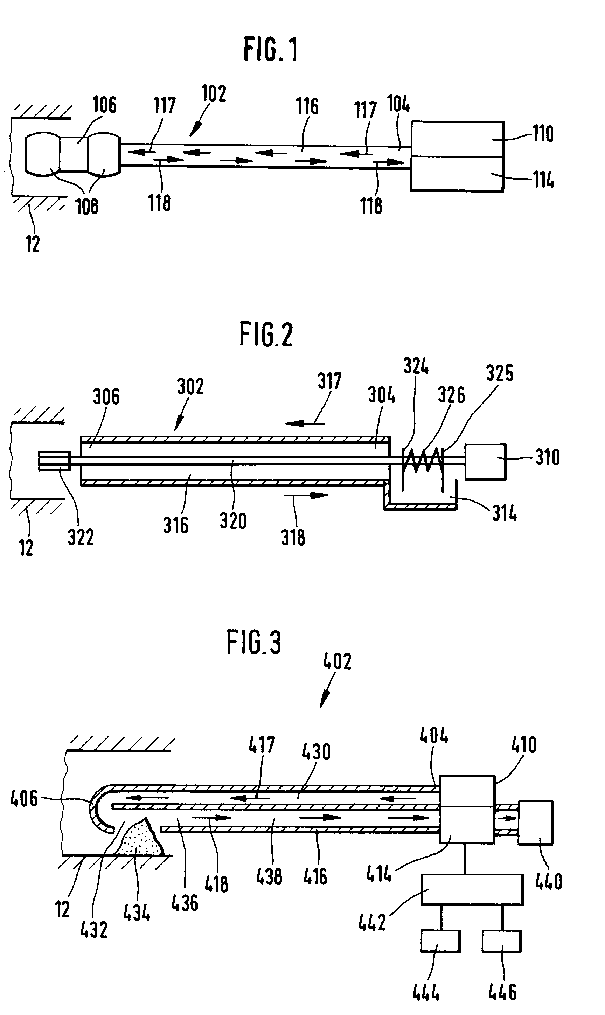

Die Erfindung betrifft therapeutische Katheter a) gemäß Fig. 1 in Form von Ballon-Kathetern (PTA, PTCA) mit einem oder mehreren expandierbaren Ballonen am distalen Endabschnitt, wobei die meßbare Energieform der Druck und/oder das Volumen einer Flüssigkeit oder eines Gases ist; b) gemäß Fig. 2 in Form von mechanischen Kathetern, deren meßbare Energieform das Drehmoment eines rotierenden Werkzeuges oder einer rotierenden Welle ist, oder Änderungen dieser Energieform in Form von Schallwellen, die im mechanischen Rotationskörper bei seiner Zusammenwirkung mit einem Patientengefäß erzeugt werden; c) gemäß Fig. 3 in Form von hydrodynamischen Kathetern, deren meßbare Energieform die kinetische Energie und/oder die Durchflußrate des Fluides und/oder die Geräuschentwicklung im Fluid beim Durchströmen des Katheters ist, wobei das Fluid eine Flüssigkeit oder ein Gas sein kann.

Fig. 1 zeigt schematisch eine Vorrichtung mit einem Ballonkatheter 102, der einen proximalen Endabschnitt 104 und einen distalen Endabschnitt 106 aufweist. Der distale Endabschnitt 106 hat einen oder mehrere axial hintereinander angeordnete, radial expandierbare Ballone 108, mit welchen Blutbahnen blockiert oder verengte Blutgefäße 12 erweitert werden können. Hierzu kann der betreffende Ballon 108 von einer am proximalen Endabschnitt 104 angebrachten Fluidquelle 110 mit Fluid, was Flüssigkeit oder Gas sein kann, expandiert werden, welches durch mindestens einen Therapie- und Informationskanal 116 des Katheters 102 hindurchströmt. Dazu wird ein Fluid bis zu einem vorbestimmten Volumen oder Druck von der Fluidquelle 110 in den betreffenden Ballon 108 gefördert. Die Fluidquelle kann eine von Hand bedienbare Kolben-Zylinder-Einheit oder ein Spritze sein. Der zum Füllen des Ballons 108 mit einem vorbestimmten Volumen erforderliche Druck hängt von dem Zustand, insbesondere dem Öffnungsquerschnitt des zu expandierenden Blutgefäßes 12 ab. Bei einem stark verengten Blutgefäß 12 ist zur Füllung des betreffenden Ballons 108 mit einem vorbestimmten Volumen ein wesentlich größerer Druck erforderlich als in einem nicht oder nur wenig verengten Blutgefäß 12. Der zeitliche Verlauf der Größe des Volumens und der zeitliche Verlauf der Größe des Druckes beim Füllen des Ballons oder der Ballone 108 können durch Sensormittel oder Meßmittel 114 am proximalen Endabschnitt 104 des Katheters 102 detektiert oder gemessen werden und geben einer erfahrenen Bedienungsperson akustisch und/oder optisch Auskunft über die Position und Wirkung der Ballone 108 im Blutgefäß 12 und über den Zustand des Blutgefäßes 12. Die Bedienungsperson kennt im Vergleich zu diesen detektierten oder gemessenen Werten die Werte eines gesunden und normal ausgebildeten Gefäßes einer vergleichbaren Person oder, falls der Patient ein Tier ist, eines vergleichbaren Tieres. Beim Füllen des Ballons oder der Ballone 108 strömt das Fluid durch den Kanal 116 in Richtung 117 zum distalen Endabschnitt 106, wohingegen beim Entleeren der Ballone 108 das Fluid in entgegengesetzter Richtung 118 durch den gleichen (oder einen anderen) Kanal 116 zum proximalen Endabschnitt 104 zurück zur Fluidquelle 110 oder zu einem Entlüftungsmittel oder Speichermittel strömt.

Fig. 2 zeigt schematisch eine Vorrichtung mit einem mechanischen Katheter 302, der mit einer sich längs durch ihn hindurcherstreckenden drehbaren Welle 320 versehen ist.

Solche mechanische Katheter 302 können insbesondere, aber nicht nur, zur Thrombektomie und zur Arterektomie verwendet werden. Die Welle 320 wird von einem Motor 310 am proximalen Endabschnitt 304 angetrieben, so daß ein am distalen Ende an ihr befestigtes Werkzeug 322 am distalen Endabschnitt 306 des Katheters in einem Gefäß 12 eines Patienten Gefäßmaterial bearbeiten, z.B. abtragen, fragmentieren oder zerstören kann. Das abgetragene oder fragmentierte Gefäßmaterial kann im Patienten verbleiben oder durch geeignete Mittel zum proximalen Endabschnitt 304 gefördert werden. Das Drehmoment der Welle 320 ist davon abhängig, wie groß der Widerstand des Gefäßes 12 im Patienten auf das Werkzeug 322 am distalen Wellenende ist. Durch einen Drehmomentsensor 314 am proximalen Endabschnitt 304 des Katheters können das jeweilige Drehmoment und Drehmomentänderungen der Welle 320 gemessen werden. Dies bedeutet, daß die Welle 320 Informationen vom distalen Endabschnitt 306 zum proximalen Endabschnitt 304 in Form von Drehmoment oder Drehmomentänderungen überträgt, welche vom Drehmomentsensor 314 gemessen und optisch und/oder akustisch angezeigt werden. Das Drehmoment kann als Drehmoment oder in anderen Maßeinheiten angezeigt werden, welche für die Bedienungsperson ein Maß für den Zustand des Gefäßes 12 und die Tätigkeit des Werkzeuges 322 in diesem Gefäß 12 sind. Der Drehmomentsensor 314 kann zwei an der Welle 320 angeordnete Winkelgeber 324 und 325 und eine zwischen ihnen angeordnete und an ihnen befestigte Torsionsfeder 326 aufweisen, mit welchen das von der Welle 320 übertragene Drehmoment und damit auch Drehmomentänderungen gemessen werden können. Die Energieübertragung zur Therapie des Patienten erfolgt auch hier durch das gleiche Element, nämlich durch die Welle 320, vom proximalen Endabschnitt 304 zum distalen Endabschnitt 306 des Katheters 302 entsprechend Pfeilen 317, und der Informationsfluß betreffend den Zustand des Gefäßes 12 und die Art und der Umfang des Einflusses des Werkzeuges 322 auf das Gefäß 12 erfolgt in entgegengesetzter Richtung entsprechend Pfeilen 318 durch die gleiche Welle 320 hindurch vom distalen Endabschnitt 306 zum proximalen Endabschnitt 304. Die Welle 320 hat somit die Funktion sowohl eines Therapieelements als auch eines Informationsübertragungselements.

Fig. 3 zeigt eine Vorrichtung mit einem hydrodynamischen Katheter 402 zur therapeutischen Behandlung eines Gefäßes 12 in einem Patienten durch ein unter Druck stehendes gasförmiges oder vorzugsweise flüssiges Fluid. Der Katheter 402 enthält einen Fluidweg, der aus einem Vorlaufkanal 430, einem zur Außenumgebung offenen Wegabschnitt 432 am distalen Endabschnitt 406 des Katheters, und einem Rücklaufkanal 438 besteht. Eine Druckfluidquelle 410 kann Druckfluid, z.B. Gas oder vorzugsweise Flüssigkeit, mit sehr hohem Druck mit beispielsweise 75 bar am proximalen Endabschnitt 404 des Katheters 402 in den Vorlaufkanal 430 in Pfeilrichtung 417 leiten. Das Druckfluid tritt am distalen Endabschnitt 406 des Katheters 402 in Form eines scharfen Fluidstrahles in den offenen Wegabschnitt 432, behandelt dort das Gefäß 12, z.B. schneidet oder fragmentiert Gefäßverengungs-Material 434, und strömt dann, unter Mitnahme des Materials 434, in das distale Ende 436 des Rücklaufkanals 438, und dann durch letzteren in Pfeilrichtung 418 zum proximalen Endabschnitt 404 zu einem Behälter 440. Am proximalen Endabschnitt 404 des Rücklaufkanals 438 ist ein Drucksensor 414 mit einem definierten hydraulischen Widerstand angeschlossen. Der Drucksensor 414 mißt den Druckabfall über dem hydraulischen Widerstand. Der gemessene Druck ändert sich in Abhängigkeit vom Strömungswiderstand des Fluides im Strömungsweg des Katheters, z.B. der Größe der Gefäßverengung 434 und davon, ob und wieviel Material 434 und gegebenenfalls Blut von dem Druckfluid aus dem Gefäß 12 des Patienten in den Rücklaufkanal 438 gefördert wird. Der vom Drucksensor 414 am proximalen Endabschnitt 404 gemessene Druck im Rücklaufkanal 438 ist deshalb eine Information darüber, ob und wie das Druckfluid auf das Gefäß 12 wirkt, und wie der Zustand des Gefäßes 12 ist, und ob der Druckfluidstrom viel, wenig oder kein Gefäßverengungsmaterial 434 transportiert, und auch Information darüber, wie der distale Endabschnitt 406 relativ zu der zu behandelnden Stelle 434 des Gefäßes 12 plaziert ist. Das gleiche Druckfluid überträgt somit über den gleichen Fluidweg 430, 432, 438 sowohl die Energie zur Therapie des Gefäßes 12 als auch Informationen für die den Katheter 402 benutzende Person.

Der Drucksensor 414 von Fig. 3 ist über eine elektronische Signal-Auswertungs-Schaltung 442 an ein optisches Anzeigegerät 444 und/oder an einen akustischen Signalgeber 446, insbesondere einen Kopfhörer oder Lautsprecher, angeschlossen. Das optische Anzeigegerät 444 zeigt dem Anwender des Katheters 402 die oben genannten Informationen. Der akustische Signalgeber 446 erzeugt in Abhängigkeit von den Informationen Töne oder Geräusche, welche der Anwender hören kann.

Die elektronische Auswertungsschaltung 442, das optische Anzeigegerät 444 und der akustische Signalgeber 446 können auch in Kombination mit den Sensoren 114 und 314 der anderen Fig. 1 und 2 verwendet werden, um deren Signale in ein optisches Anzeigesignal und/oder in ein akustisches Anzeigesignal umzuwandeln.

Bei den Ausführungsformen nach den Fig. 1 und 3 kann das Fluid anstelle durch einen Kanal durch mehrere Kanäle parallel hindurchgeleitet werden.

In dem Druckfluid des Katheters 102 von Fig. 1 und des Katheters 402 von Fig. 3 entstehen Geräusche in Abhängigkeit davon, ob das Druckfluid schnell oder langsam strömt, ob es Teilchen des Gefäßes 12 fragmentiert, und/oder ob sich sein Druck ändert. Diese Geräusche sind Informationen über den Zustand des Gefässes 12 und über die Wirkung des Druckfluides am distalen Endabschnitt des Katheters. In ähnlicher Weise entstehen in der Welle 320 des Katheters 302 von Fig. 2 Geräusche, und damit Informationen in Abhängigkeit von dem Zustand des Gefäßes 12 und der Wirkung des Werkzeuges 322 am distalen Endabschnitt des Katheters. Deshalb bestehen bevorzugte Ausführungsformen darin, daß der Sensor 114 von Fig. 1, der Sensor 314 von Fig. 2, und der Sensor 314 von Fig. 3 Geräusch- oder Schallsensoren sind und Mittel zur optischen und/oder akustischen Anzeige der Informationen aufweisen, vorzugsweise Lautsprecher oder Kopfhörer.

Bei allen Ausführungsformen können die Informationen automatisch in elektronischen oder anderen Speichermitteln registriert und gespeichert werden.

## Patentansprüche

1. Vorrichtung mit einem therapeutischen Katheter zur therapeutischen Behandlung von Gefäßen in einem Patienten, wobei der Katheter einen distalen Endabschnitt aufweist, der in ein Patientengefäß einsetzbar ist, und einen Fluidkanal (116; 416) für gasförmiges oder flüssiges, unter Druck stehendes Fluid aufweist, welches sich vom proximalen Endabschnitt zum distalen Endabschnitt oder vom proximalen Endabschnitt zum distalben Endabschnitt und wieder zurück zum proximalen Endabschnitt erstreckt und am distalen Endabschnitt Mittel (108; 432) zur Behandlung eines Patientengefäßes (12) aufweist,
**dadurch gekennzeichnet,**
daß am proximalen Endabschnitt Sensor- oder Meßmittel (114; 414) an den Fluidkanal (116; 416) angeschlossen sind, welche in Abhängigkeit von einem oder mehreren physikalischen Werten oder Wertänderungen des Fluides in Abhängigkeit von externen Einwirkungen auf das Fluid am distalen Endabschnitt des Katheters ein optisch oder akustisch erkennbares Signal oder einen optisch oder akustisch erkennbaren Meßwert erzeugen, welches oder welcher für eine Bedienungsperson des Katheters ein Maß für die Situation ist, welcher das Fluid in der Umgebung des distalen Endabschnitts des Katheters ausgesetzt ist.

2. Vorrichtung nach Anspruch 1,
**dadurch gekennzeichnet,**
daß im therapeutischen Katheter (402) ein Vorlaufkanal (430) und ein Rücklaufkanal (438) für das Fluid gebildet sind, welche am distalen Endabschnitt des Katheters durch einen zur externen Umgebung offenen Strömungsweg-Abschnitt (432) miteinander verbunden sind, auf welchem das Fluid die Umgebung des distalen Endabschnittes des Katheters kontaktieren kann.

3. Vorrichtung nach Anspruch 2,
**dadurch gekennzeichnet,**
daß das stromabwärtige Ende des Vorlaufkanals (430) als Düse ausgebildet ist, die auf einen ihr gegenüberliegenden Einlaß (436) des Rücklaufkanals (438) gerichtet ist und das unter hohem Druck zugeführte Fluid in einen scharfen Fluidstrahl formt, welcher auf dem offenen Strömungsweg-Abschnitt (432) Gefäßmaterial eines Patienten fragmentieren und in den Rücklaufkanal (438) fördern kann.

4. Vorrichtung nach Anspruch 2 oder 3,
**dadurch gekennzeichnet,**
daß das Sensor- oder Meßmittel (114; 414) einen Drucksensor oder Schallsensor zum Messen von Drücken oder von Geräuschen des Fluides aufweist, welche am distalen Endabschnitt des Katheters erzeugt werden.

5. Vorrichtung nach Anspruch 1 oder 4,
**dadurch gekennzeichnet,**
daß der therapeutische Katheter (102) an seinem distalen Endabschnitt (106) mindestens ein Expansionselement (108) aufweist, welches durch das Fluid expandierbar ist, daß mindestens ein Fluidkanal (116) für das Fluid vorgesehen ist, welcher sich vom proximalen Endabschnitt (104) zum distalen Endabschnitt (106) durch den Katheter (102) erstreckt und am proximalen Endabschnitt (104) an eine Druckfluidquelle (110) und an die Sensor- oder Meßmittel (114) angeschlossen ist, welche auf Druck oder Geräusche oder Volumen des Fluides ansprechen, welches dem mindestens einen Expansionselement (108) zugeführt wird.

6. Vorrichtung mit einem therapeutischen Katheter zum therapeutischen Behandeln von Gefäßen (12) in einem Patienten, wobei der Katheter einen distalen Endabschnitt aufweist, der in ein Patientengefäß einsetzbar ist, und einen Rotationskörper (322) am distalen Endabschnitt aufweist, welcher zur mechanischen Behandlung des Patientengefäßes (12) von einer Welle (320) antreibbar ist, die sich in Längsrichtung durch den Katheter erstreckt und am proximalen Endabschnitt des Katheters von einem Antriebsmittel (310) antreibbar ist,
**dadurch gekennzeichnet,**
daß die Welle (320) mit einem Sensormittel oder Meßmittel (314) versehen ist, welches die Energie und/oder Energieänderungen detektiert oder mißt, welche in der rotierenden Welle in Abhängigkeit von Einwirkungen der externen Umgebung (12) auf den Rotationskörper (322) entstehen.

7. Vorrichtung nach Anspruch 6,
**dadurch gekennzeichnet,**
daß die Sensormittel oder Meßmittel (314) das Drehmoment der angetriebenen Welle (320) detektieren oder messen.

8. Vorrichtung nach einem der Ansprüche 1 bis 7,
**dadurch gekennzeichnet,**
daß die Sensormittel oder Meßmittel (114; 314; 414) einen akustischen Signalgeber (446) aufweisen, welcher die detektierten oder gemessenen Werte in akustische, für Personen hörbare Signale umwandelt.

9. Vorrichtung nach einem der Ansprüche 1 bis 8,
**dadurch gekennzeichnet,**
daß die Sensormittel oder Meßmittel (114; 314; 414) ein optisches Anzeigeelement (444) zur Umwandlung und Anzeige des detektierten und gemessenen Wertes aufweisen.

10. Vorrichtung nach einem der Ansprüche 1 bis 9,
**dadurch gekennzeichnet,**
daß das Sensormittel oder Meßmittel (114; 314; 414) eine Vorrichtung aufweist, welche im Therapie-Fluid oder in der Welle eines Therapie-Rotationskörpers Schallwellen detektiert, die durch externe Einflüsse auf das Fluid oder den Rotationskörper entstehen, und mindestens einen Lautsprecher oder Kopfhörer aufweist, welcher diese Schallwellen für ein menschliches Ohr hörbar macht.
